# EUROPÄISCHE PATENTSCHRIFT

(11) **EP 0 681 446 B1**
(45) Veröffentlichungstag und Bekanntmachung des Hinweises auf die Patenterteilung: **02.02.2000**
(21) Anmeldenummer: 95902029.8
(22) Anmeldetag: 28.11.1994
(51) Int. Cl.: A61B 1/00, A61B 1/04

(54) **ENDOSKOPISCHES INSTRUMENTARIUM**
ENDOSCOPIC INSTRUMENTARIUM
INSTRUMENT ENDOSCOPIQUE

(30) Priorität: 29.11.1993 DE 4340501
(43) Veröffentlichungstag der Anmeldung: 15.11.1995
(73) Patentinhaber: ETB ENDOSKOPISCHE TECHNIK GMBH BERLIN, D-12555 Berlin (DE)
(72) Erfinder: LASER, Helmut, D-10405 Berlin (DE); HAUER, Gerald, D-82362 Weilheim (DE)
(74) Vertreter: Pfeiffer, Rolf-Gerd, Dipl.-Phys.
(86) Internationale Anmeldenummer: DE9401401
(87) Internationale Veröffentlichungsnummer: WO9514425

(56) Entgegenhaltungen:
- EP-A- 0 369 936
- EP-A- 0 497 347
- EP-A- 0 585 826
- FR-A- 2 212 132
- US-A- 3 481 325

## Beschreibung

Die Erfindung betrifft ein endoskopisches Instrumentarium, insbesondere ein Instrumentarium für die endoskopische subfasciale Discision der Perforansvenen (ESDP).

Zwischen 10 und 15% der Erwachsenen leiden unter einer ausgeprägten Varikosis an den unteren Extremitäten, bis zu 16% dieser Patienten haben insuffiziente Perforansvenen. Im Rahmen der Therapie der primären Varikosis und posttrhrombotisch oder varikös bedingter trophischer Hautstörungen werden diese insuffizienten Perforansvenen subfascial ausgeschaltet. Dabei erforderten die bisherigen operativen Verfahren eine ausgedehnte Freilegung der Perforansvenen und waren häufig mit postoperativen Wundheilungsstörungen behaftet oder wiesen den Mangel geringerer Präzision auf.
Im Rahmen der Minimal Invasiven Chirurgie wurde von Dr. G. Hauer die Technik der Endoskopischen Subfascialen Discision der Perforansvenen (ESDP) entwickelt, die in ihrem bisherigen Stand als das bislang effektivste therapeutische Prinzip gilt.
Das im genannten Zusammenhang entstandene Instrumentarium (vgl. u.a. Jugenheimer, M; Junginger, Th.: PHLEBOLOGIE, 4. Jahrgang 8/92, S. 540 ff.) umfaßt Operationskaltlichttuben unterschiedlicher Durchmesser, ein herkömmliches Operationslaparoskop und Hilfsinstrumente, wie bipolare Koagulationszangen und endoskopische Scheren.
Diese Standardkomponenten aus unterschiedlichen Bereichen der Endoskopie wurden für das Verfahren der ESDP nur geringfügig modifiziert, so daß das Instrumentarium zwangsläufig noch eine Reihe wesentlicher Mängel aufweist.

Eine nachträglich angebrachte Verriegelung der Instrumente in dem OP-Laparoskop hat sich im praktischen Einsatz nicht bewährt. Für die gleichzeitige Handhabung von Tubus, Endoskop und Arbeitsinstrument ist die helfende Hand eines Assistenten notwendig. Für die mit einem relativ hohen Kraftaufwand verbundene Manipulation des Tubus wird dessen Griff außerdem als zu instabil empfunden. Einen wesentlichen Mangel stellt die unzureichende Qualität des optischen Systems des OP-Laparoskops dar, dessen geringe Apertur bei den schlechten Beleuchtungsbedingungen im OP-Situs (hohe Absorption durch starke Blutungen) eine Wiedergabe über übliche CCD-Endokameras bisweilen in Frage stellte. Die bei Operationslaparoskopen übliche Blickrichtung von 5...10° ist für die Sicht auf das eingeführte Instrument in der Regel nicht ausreichend. Die gerätebedingte Abführung der Kabel für Kamera, HF-Instrumente, faseroptische Beleuchtung und der Absaugung von Koagulationsgasen in unterschiedlichen Richtungen zur Instrumentenachse erschwert die Handhabung zusätzlich.

Aufgabe der Erfindung ist die Schaffung eines endoskopischen Instrumentariums, insbesondere für die ESDP, das der medizinischen Verfahrensweise bestmöglich angepaßt ist und die genannten Mängel der bisherigen Instrumentarien nicht aufweist.

Eine Lösung wurde z.B. bei einem Laparoscop in EP-0 497 347 beschrieben.

Die Aufgabe wird durch die Merkmale des ersten Patentanspruchs gelöst.

Die Erfindung soll anhand nachstehenden Ausführungsbeispiels näher erläutert werden. Es zeigen:

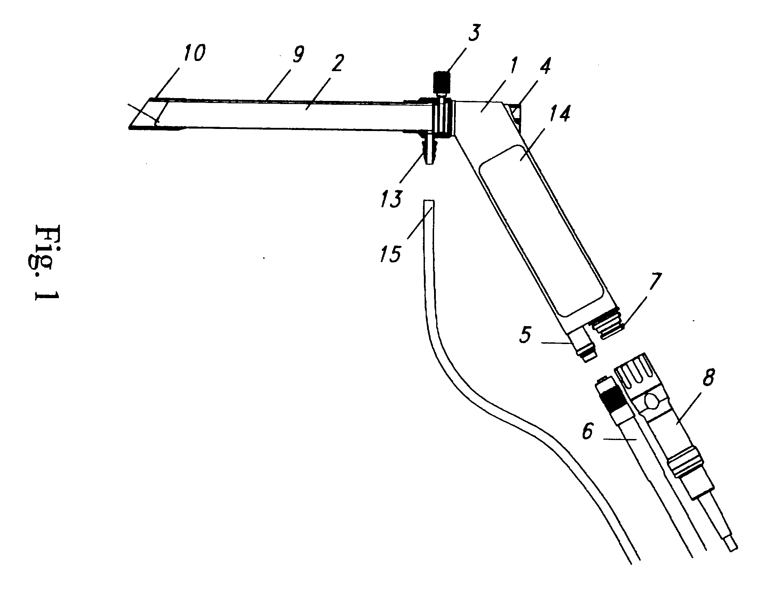

- Fig. 1: eine Seitenansicht erfindungsgemäßen endoskopischen Instrumentariums im teilweisen Schnitt und

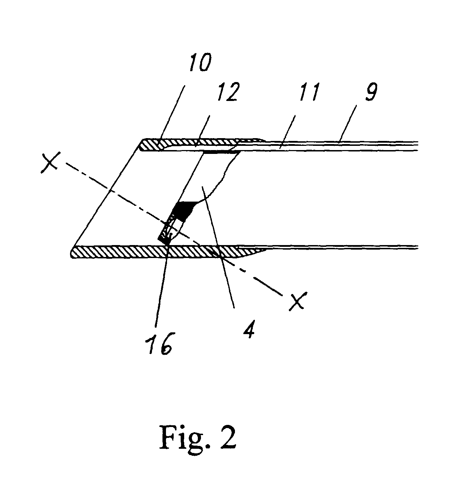

- Fig. 2: eine vergrößert dargestellte, teilweise geschnittene schematische Darstellung des distalen Endes eines endoskopischen Instrumentariums gemäß Fig. 1.

Das erfindungsgemäße Instrumentarium besteht aus einem speziell ausgebildeten Operationsendoskop 1, auf dessen einführbaren Schaft 2 ein Tubus 9 mittels einer Schraube 3 oder einer anderen geeigneten Befestigung fest verriegelbar ist. Der Schaft 2 des Operationsendoskops enthält ein optisches System zur Bildaufnahme und Übertragung, nicht näher dargestellte faseroptische Lichtleiter zur Übertragung des Lichtes einer externen Kaltlichtquelle zum OP-Situs und einen Arbeitskanal 4 zur Einführung chirurgischer Instrumente, wie endoskopischer Messer, Scheren oder Instrumente der chirurgischen HF-Chirurgie. Ein Handgriff 14 am proximalen Ende des Instruments erlaubt die Handhabung des Instruments mit dem dazu notwendigen Kraftaufwand. Das Beleuchtungssystem und das abbildende optische System sind nach einer Abwinklung durch das Innere des Handgriffes 14 zu dessen Ende geführt, wo an einem Stutzen 5 der Anschluß eines Kabellichtleiters 6 der externen Kaltlichtquelle und an einer Kupplung 7 der Anschluß einer Kamera 8 zur Übertragung des Bildes auf einen Monitor erfolgt. Ebenso liegt es im Rahmen der Erfindung, z.B. zum Einsatz gelangende CCD-Kameras mit in genanntem Handgriff zu integrieren.
Auf dem Schaft 2 des Operationsendoskops ist der Tubus 9 fest verriegelbar. Das distale Ende des Tubus 9 ist als verdickte, atraumatisch gestaltete Lippe 10 ausgebildet und ragt über das Ende des Schaftes des Operationsendoskops hinaus. Durch eine bevorzugt unterschiedliche Durchmesserfestlegung von Schaftaußendurchmesser und Tubusinnendurchmesser ist ein Zwischenraum 11 gebildet, durch den, bspw. bei einer HF-Koagulation entstehende Verbrennungsgase o.ä. abgesaugt werden können. Das distale Ende des Schaftes wird im Inneren der Lippe 10 spielarm geführt. Es ist ein Kanal 12 im Inneren der Lippe auf der Seite des Arbeitskanals des Operationsendoskops vorgesehen, der ein zum Absaugen notwendiges Lumen als Verbindung zum Rohrzwischenraum 11 schafft. Dabei ist erfindungsgemäß der Kanal 12 diametral zu einer Frontlinse 16 angeordnet und der Zwischenraum 11 ist durch eine exzentrische Anordnung von Schaft 2 und Tubus 9 im wesentlichen auf der Seite des Kanals 12 festgelegt. Ein Schlauchstutzen 13 zum Ansatz eines Saugschlauches 15 befindet sich in einer Ebene mit und in Richtung des Handstückes 14, wodurch Behinderungen des Operateurs äußerst gering gehalten sind. Die optische Achse X-X einer Frontlinse bzw. eines Linsensystems 16 ist im Rahmen der Erfindung in einem Winkel zur Mittenachse des Schaftes 2 in einer Größenordnung von 30° festgelegt.

Das erfindungsgemäße Instrumentarium ist zwar vornehmlich für das Verfahren der endoskopischen subfascialen Discision der Perforansvenen (ESDP) konzipiert, jedoch nicht darauf beschränkt. Unter Beibehaltung des selben erfindungsgemäßen Aufbaus läßt sich durch einfache geometrische Anpassungen, bspw. eine Durchmesservergrößerung genannten Schaftes und Tubus, ein Instrumentarium auch für andere endoskopische Einsatzgebiete schaffen.
Für das Verfahren der ESDP steht durch vorliegende Erfindung ein Instrumentarium zur Verfügung, das den Zugang fernab trophisch gestörter Bereiche ermöglicht, wodurch Wundheilstörungen wirkungsvoll vermieden werden. Unter endoskopischer Sicht kann die Unterschenkelfaszie vollständig und ohne Begleitverletzungen gespalten werden. Die medialen und dorsalen Gruppen der Perforansvenen des Unterschenkels lassen sich zuverlässig auffinden und durchtrennen; dies auch, wenn eine genaue präoperative Lokalisierung bei bestehendem Ulcus cruris nicht möglich ist.

### Bezugszeichenliste

- 1 -: Operationsendoskop
- 2 -: Schaft
- 3 -: Schraube
- 4 -: Arbeitskanal
- 5, 7 -: Koppelstellen
- 8 -: Kamera
- 9 -: Tubus
- 10 -: Lippe
- 11 -: Zwischenraum
- 12 -: Kanal
- 13 -: Schlauchstutzen
- 14 -: Handstück
- 15 -: Saugschlauch
- 16 -: Frontlinse bzw. Linsensystem
- X-X -: optische Achse der Linsen 16

## Patentansprüche

1. Endoskopisches Instrumentarium bestehend aus einem Operationsendoskop (1) und einem Tubus (9), wobei das Operationsendoskop (1) ein optisches System zur Bildaufnahme und -weiterleitung, ein faseroptisches Beleuchtungssystem und einen Arbeitskanal (4) enthält, am proximalen Ende des Instruments ein seitlicher, in einem Winkel abgehender Handgriff (14) vorgesehen ist, in dessen Inneren zumindest ein Abbildungs- und ein Beleuchtungssystem eingebracht und bis zu dessen Ende zu Koppelstellen (5, 7) geführt ist, das distale Ende des Tubus (9) das distale Ende des Operationsendoskops (1) überragt und in Form einer dickeren atraumatisch gestalteten Lippe (10) ausgebildet ist und diese einen Kanal (12) umfaßt, welcher diametral zu einer Frontlinse, bzw. einem Linsensystem, (16) angeordnet und über einen Zwischenraum (11) zwischen einem Schaft (2) und Tubus (9), wobei der Tubus (9) fest -d.h. in einer vorgebbaren Lage arretiert- auf dem Schaft (2) des Endoskops (1) verriegelbar ist und das distale Ende des Schaftes (2) im Inneren der Lippe (10) spielarm geführt ist, mit einem Anschlußstutzen (13) in Verbindung gebracht ist, wobei der Anschlußstutzen (13) im wesentlichen in einer Ebene mit und in Richtung des Handstückes (14) geführt ist.

2. Endoskopisches Instrumentarium nach Anspruch 1., dadurch gekennzeichnet, daß die optische Achse (X-X) der Frontlinse, bzw. des Linsensystems (16) zur Mittenachse des Schaftes (2) in einem Winkel in der Größenordnung von 30° festgelegt ist.

3. Endoskopisches Instrumentarium nach Anspruch 1., dadurch gekennzeichnet, daß eine zur Bildaufnahme dienende Kamera (8), bevorzugt eine CCD-Kamera, mit in den Handgriff (14) integriert ist.

## Claims

1. An endoscopic device constituted of a surgical endoscope (1) and a tube (9), said surgical endoscope (1) comprising an optical system for detecting and transmitting images, a fiber-optical illumination system and an operation channel (4), and being laterally provided at its proximal end portion with a handle (14) including an angle, said handle (14) being provided with at least one imaging and illumination system in its interior and extending therein to its end portion to coupling means (5, 7), a distal end portion of said tube (9) projecting beyond the distal end portion of said surgical endoscope (1) and being embodied as a bulgy a-traumatic lip (10) including a channel (12) arranged in diametrical opposition to a front lens and a lens system (16), respectively, and being adapted to be connected to a coupling means (13) via an intermediate space (11) between a shaft (2) and said tube (9), and whereby said coupling means (13) extends substantially in a same plane as and in direction of said handle (14), and whereby said tube (9) being adapted to be undisplaceably - that is, arrested in a preselectable position - locked onto the shaft (2) of the endoscopic device (1) and the distal end of the shaft (2) is guided at low backlash within the interior of said lip (10).

2. An endoscopic device as claimed in claim 1, wherein an optical axis (X-X) of said front lens and said lens system (16), respectively, include an angle in an order of size of 30° relative to a central axis of said shaft (2).

3. An endoscopic device as claimed in claim 1, wherein a camera (8) for image detection, preferably a CCD-camera, is also installed within said handle (14).

## Revendications

1. Instrument endoscopique composé d'un endoscope opératoire (1) et d'un tube (9); l'endoscope opératoire (1) comprend un système optique pour la prise et la transmission d'images, un système d'éclairage fonctionnant avec des fibres optiques et un canal opératoire ; sur l'extrémité proximale de l'instrument est disposée une manette (14) sortant du tube de manière angulaire et comprenant dans son intérieur au moins un système optique et un système d'éclairage qui s'étendent jusqu'au bout de la manette où sont situés les points d'accouplement (5,7); l'extrémité distale du tube (9) qui dépasse l'extrémité distale de l'endoscope opératoire (1) est conçue comme une lèvre (10) assez épaisse pour permettre une exploration non traumatisante , la lèvre, dont la position est diamétralement opposée à une lentille frontale ou un système de lentilles( 16), est logée autour d'un canal (12), qui traversant l'interstice (11) qui sépare la tige (2) et le tube (9), le tube pouvant être monté fixe - c'est-à-dire immobilisé dans une position prédéterminée; peut être verrouillé sur la tige (2) de l'endoscope (1) tandis que l'extrémité distale de la tige (2) se déplace de manière guidée et sans jeu à l'intérieur de la lèvre (10) et se branche sur le raccord (13), lequel se déplaçant au même plan et dans le même sens que la manette (14).

2. L'instrument endoscopique selon la revendication 1 est caractérisé en ce que l'axe optique (X-X) de la lentille frontale ou du système de lentilles (16) est disposé par rapport à l'axe médian de la tige (2), de manière qu'il forme avec celui-ci angle de près de 30°.

3. L'instrument endoscopique selon la revendication 1 est caractérisé en ce qu'une caméra (8) destinée à la prise d'images, de préférence une caméra CCD, est également intégrée dans la manette.
